# EUROPEAN PATENT SPECIFICATION

(11) **EP 1 135 104 B1**
(45) Date of publication and mention of the grant of the patent: **21.02.2007**
(21) Application number: 99964001.4
(22) Date of filing: 29.11.1999
(51) Int. Cl.: A61Q 19/00, A61K 8/02, A61K 8/37

(54) **TISSUE PAPER PRODUCT, AND PROCESS FOR MAKING THE PRODUCT**
Papiertaschentuchprodukt UND VERFAHREN ZUR HERSTELLUNG DES PRODUKTES
PAPIERS-MOUCHOIRS ET LEUR PROCEDE DE FABRICATION

(30) Priority: 01.12.1998 EP 98122815
(43) Date of publication of application: 26.09.2001
(73) Proprietor: THE PROCTER & GAMBLE COMPANY, Cincinnati, Ohio 45202 (US)
(72) Inventor: VON HEIMBURG, Joachim, D-61348 Bad Homburg (DE); PALUMBO, Gianfranco, D-61348 Bad Homburg (DE)
(74) Representative: Kremer, Véronique Marie Joséphine
(86) International application number: PCT/US1999/028180
(87) International publication number: WO 2000/032164

(56) References cited:
- EP-A- 0 117 613
- EP-A- 0 564 307
- EP-A- 0 875 233

## Description

The present invention relates to tissue paper products comprising a tissue paper substrate and an enzyme inhibiting composition, and to a process for making the product.

Tissue paper products have been disclosed which comprise a substrate and a soothing lotion. US-A-4 513 051, issued on 23^{rd} April 1985, and US-A-5 525 345, issued on 11^{th} June 1996, disclose such tissue paper products comprising lotion for use as facial tissues and toilet tissues. Tissues are rubbed against the skin when used and this can cause a painful raw or red skin to develop. This is particularly the case when someone is afflicted with a cold and needs to blow their nose frequently. The soothing lotion is intended to reduce the rawness and redness of the skin by making the surface of the tissue paper substrate less harsh and sometimes by also applying an emollient to the skin.

EP-A-0 117 613, published on 5^{th} September 1984, discloses agents for the treatment and prophylaxis of diaper rash and diaper dermatitis. The disposable absorbent articles which are disclosed are diapers, incontinence pads and wipes. Various agents are disclosed including petrolatum, zinc oxide and other metal salts, and triacetin.

In EP-A-0 564 307, Levi, S., discloses an antiskin nash preparation, that prevent or at least substantially inhibit the development of unhealthy skin conditions resulting from contact with excreted body fluids, by inhibiting the activity of enzymes present in such fluids, e.g urease in case of urine.

The object of the present invention is to provide a tissue paper product, which comprises a tissue paper substrate, which reduces painful skin conditions, in particular in the nasal area by providing a tissue paper product that is a facial handkerchief which delivers an active agent on to the skin when it is used.

### Summary of the Invention

The object of the invention is achieved by means of an enzyme inhibiting composition, the enzyme inhibiting composition being transferable from the tissue paper substrate onto the skin and being present in an amount sufficient to inhibit enzyme activity upon the skin. Preferably the tissue paper product comprises a tissue paper substrate and a topically applied lotion, the topically applied lotion comprising the enzyme inhibiting composition. The tissue paper of the present invention is a facial handkerchief.

In a preferred process, the tissue paper product of the present invention is made by a wet-laying process.

### Detailed Description of the invention

The tissue paper substrate useful in the present invention may be made by common methods well-known to the person skilled in the art, such as by dewatering suitable pulp fumishes using, for example, papermakers felt. This process may be carried out in batch, but commercially it is usually carried out on continuous papermaking machines. Highly preferred tissue paper may be made by the processes described in US-A-3 301 746, published on January 31^{st} 1967, or EP-A-0 140 404, published on May 8^{th} 1985. Paper made according to EP-0 140 404 is composed of first and second regions, the first region having a macroscopically monoplanar, patterned, continuous network having a high density and a low basis weight relative to the second region, and the second region being composed of a high basis weight and plurality of discrete domes having low densities relative to the first region. The preferred average density of the first, network, region is from 0.4 to 0.8 gram per cubic meter and the average density of the domes of the second region is from 0.04 to 0.15 gram per cubic centimeter. Optional steps for the process of making the web, or for subsequent processing, include foreshortening, or creping; embossing and printing.

A suitable pulp furnish for the process of making the tissue paper substrate preferably contains papermaking fibres consisting essentially of cellulose fibres (commonly-known as wood pulp fibres) or cellulose-derived fibres (including, for example, rayon, viscose). Fibres derived from soft woods (gymnosperms or coniferous trees) and hard woods (angiosperms or deciduous trees) are contemplated for use in this invention. The particular species of tree from which the fibres are derived is immaterial. The wood pulp fibers can be produced from the native wood by any convenient pulping process. Chemical processes such as sulfite, sulphate (including the Kraft) and soda processes are suitable. Mechanical processes such as thermochemical (or Asplund) processes are also suitable. In addition, the various semi-chemical and chemi-mechanical processes can be used. Bleached as well as unbleached fibers are contemplated for use.

The tissue paper product of the present invention preferably has a basis weight of from 10 to 100 grams per square metre, more preferably from 25 to 60 grams per square meter, and may, optionally, consist of multiple plies. Preferably the tissue paper product consists of between 2 and 4 plies. The term "tissue paper substrate" as used herein refers to one or more plies of tissue paper.

By "enzyme inhibiting composition" what is meant herein is any composition which comprises an active component to inhibit enzyme activity on the human skin. Preferred active components of enzyme inhibiting compositions include lipase inhibitors, protease inhibitors, and mixtures thereof.

Painful or irritating "red nose" conditions are reduced or alleviated by transferring on to the nasal area an enzyme inhibiting composition which inhibits the activity of one or more enzymes which are present in human mucus. Similarly painful or irritating conditions caused by damaged or weakened skin in the perianal region are reduced or alleviated by transferring an enzyme inhibiting composition to the perianal region which inhibits the activity of one or more enzymes which are present in human faeces or urine. Particularly suitable active components of enzyme inhibiting compositions may be selected from the ester compounds, including:
(a) a triester compound of the formulation: 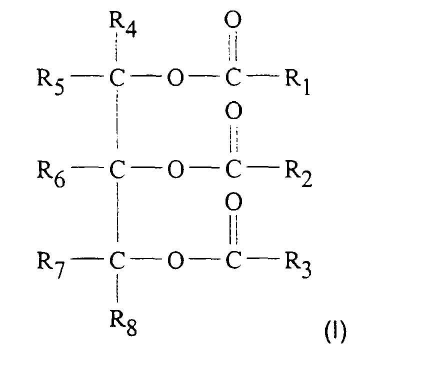
wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, alkenyl or hydroxyalkyl groups, hydroxy, chloride, bromide, amine or hydrogen ;

A highly preferred additional ester compound for use in, or for preparation of the compositions of the invention is of the formulation: 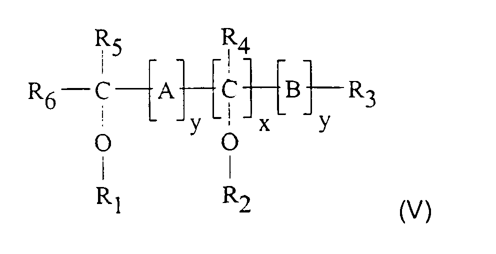 or 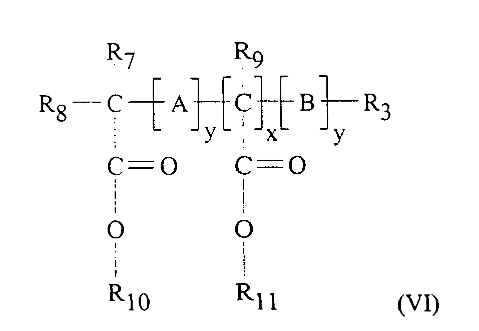 wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃ R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁-C₆ linear or branched alkylene, alkenylene, alkoxylene, hydroxyalkylene groups; the values of x are independently from 0 to 15; the values of y are independently 0 or 1, with the proviso that when x =2 and y=0, at least one R₂ is an alkyl, alkenyl, arylalkyl, hydroxyalkyl group with from 1 to 24 carbon atoms or hydrogen

It should be understood that for the purpose of this invention, the groups R₁-R₁₁ of formulations (V) and (VI) above can be substituted by any appropriate substituent group.

Preferred are the ester compounds as defined above, wherein the compound is of formula (V) or (VI) wherein x is 1 or 2, y is 0; R₁ and one R₂ are a C₂-C₁₆ acyl group, R₁₀ and one or more R₁₁ are a C₂-C₁₆ alkyl group; R₃, R₄, R₅, R₆, R₇ and R₈ are hydrogen

It is highly preferred that the additional ester compound is a mono or diester of formula (VI), most preferably a mono or diester of citric acid or tartaric acid (or salts thereof), or a triester of citric acid.

Another highly preferred component of the enzyme inhibiting composition is triacetin. Triacetin, (1,2,3-propanetriol triacetate), is a colourless, oily liquid which is known as a topical anti-fungal. The Merck Index, 9^{th} Edition, page 1232, Merck and Co. (1976). The "self-regulating" action of triacetin is known, i.e. it is known that at the neutral or higher pH of the affected skin glycerol and free fatty acid (acetic acid) are rapidly liberated from triacetin as a result of the action of the esterase enzymes found abundantly in skin, serum, and fungi. The growth of the fungi is inhibited by the free fatty acid.

The enzyme inhibiting composition is present in an amount sufficient to inhibit enzyme activity upon the skin, preferably from 0.05% to 10%, and more preferably from 0.5% to 5%, by weight of the tissue paper product.

The preferred process for the manufacture of the tissue paper substrate is a wet laying process comprising the steps of:
wet-laying a pulp to form a web, the pulp containing fibres consisting essentially of cellulose fibres or cellulose-derived fibres;
drying the web to form a ply of a tissue paper substrate;
optionally combining two or more plies to provide the tissue paper substrate;
topically applying an enzyme inhibiting composition, the enzyme inhibiting composition being transferable from the tissue paper substrate onto the skin and being present in an amount sufficient to inhibit enzyme activity upon the skin.

One preferred way of topically applying the enzyme inhibiting agent is to apply it to the surface of the tissue paper substrate, for example in the form of a lotion. In addition to the enzyme inhibiting composition, the lotion may optionally also comprises softening/debonding agents, emollients, immobilizing agents and mixtures thereof. Suitable softening/debonding agents include quaternary ammonium compounds, polysiloxanes, and mixtures thereof. Suitable emollients include propylene glycol, glycerine, triethylene glycol, spermaceti or other waxes, fatty acids, fatty alcohols and fatty alcohol ethers having from 12 to 28 carbon atoms in their fatty acid chain, and mixtures thereof. Suitable immobilizing agents include polyhydroxy fatty acid esters, polyhydroxy fatty acid amides and mixtures thereof. Other optional components include perfumes, antibacterial actives, antiviral actives, disinfectants, pharmaceutical actives, film formers, deodorants, opacifiers, astringents, solvents and the like.

One preferred way of topically applying the enzyme inhibiting agent or lotion is to apply it to the surface of the tissue paper substrate by any of a number of conventional methods including spraying, printing (for example, flexographic printing), coating (for example, gravure coating), extrusion or combinations of these application techniques, for example spraying the lotion on to a rotating surface, such as a calendar roll, that then transfers the composition to the surface of the tissue paper substrate. The lotion composition can be applied to one surface of the tissue paper substrate, or both surfaces. Preferably, the lotion is applied to both surfaces of the paper web.

The manner of applying the lotion composition to the tissue paper web should be such that the web does not become saturated with the lotion composition. If the web becomes saturated with the lotion composition, there is a greater potential for debonding of the paper to occur, thus leading to a decrease in the tensile strength of the paper. Also, saturation of the paper is not required to obtain the softness and lotion-like feel benefits from the lotion composition.

### Examples

### Example 1

A four-ply tissue paper product is made having a total basis weight of 60.4 g/m² for the product, which is a facial handkerchief. The tissue paper plies are made by removing water from a pulp furnish using a commercially available paper making machine. The furnish composition is based on 40% Northern Softwood Kraft long fibre pulp (provenience Soedra, chlorine free bleached) and 60% hard wood fibre (Eucalyptus, provenience Ponte Vedra, chlorine free bleached). The paper is creped and dried to 5.5% of residual moisture. The tensile strength in the machine direction is about 23 Newtons per 50 millimetres, and the tensile strength in the cross-machine direction is about 12 Newtons per 50 millimetres. Four essentially identical plies, each with a basis weight of 15.1 g/m², are converted to form the four-ply tissue paper substrate.

Onto this substrate a lotion is printed from both outer surface sides. The total amount of lotion printed onto the tissue is 4.5% of its dry weight.

The composition of the lotion is: 3.8% by weight of triacetin, 7% by weight of a quaternised fatty acid ester (C18 alkyl), 3.5% by weight of alkylpolyglycol (Alkyl being C2, MW 18 - 20000), 0.1% by weight of silicon oil defoamer of low viscosity, and water to balance to 100%.

## Claims

1. A tissue paper product that is a facial handkerchief comprising a tissue paper substrate and an enzyme inhibiting composition, the enzyme inhibiting composition being transferable from the tissue paper substrate onto the skin and being present in an amount sufficient to inhibit enzyme activity upon the skin, and wherein the enzyme inhibiting composition comprises inhibitors of enzymes present in human mucus.

2. A tissue paper product according to claim 1 comprising a tissue paper substrate and a topically applied lotion, the topically applied lotion comprising the enzyme inhibiting composition.

3. A tissue paper product according to claim 3 wherein the enzyme inhibiting composition comprises inhibitors selected from the group consisting of lipase inhibitors, protease inhibitors, and mixtures thereof.

4. A tissue paper product according to any of claims 1 to 4 wherein the enzyme inhibiting composition comprises one or more ester compounds.

5. A tissue product according to claim 2 wherein the topically applied lotion further comprises a softening agent selected from the group consisting of quaternary ammonium compounds, polysiloxanes and mixtures thereof.

6. A tissue paper product according to any of the previous claims wherein the tissue paper product has a basis weight of from 10 to 100 grams per square metre, preferably from 25 to 60 grams per square meter.

7. Use of an enzyme inhibiting agent in the manufacture of a facial handkerchief for the inhibition of enzyme activity upon the skin of the nasal region.

8. A process for the manufacture of a tissue paper product that is a facial handkerchief comprising the steps of:
wet-laying a pulp to form a web, the pulp containing fibres consisting essentially of cellulose fibres or cellulose-derived fibres;
drying the web to form a ply of a tissue paper substrate;
optionally combining two or more plies to provide the tissue paper substrate;
topically applying a lotion;
**characterised in that** the lotion comprises an enzyme inhibiting composition, the enzyme inhibiting composition being transferable from the tissue paper substrate onto the skin and being present in an amount sufficient to inhibit enzyme activity upon the skin, and
wherein the enzyme inhibiting composition comprises inhibitors of enzymes present in human mucus.

9. A process according to claim 9 wherein the lotion is either sprayed or printed on to the tissue paper substrate.

## Patentansprüche

1. Tissue-Papierprodukt, das ein Taschentuch ist, umfassend ein Tissue-Papiersubstrat und eine Enzymhemmungszusammensetzung, wobei die Enzymhemmungszusammensetzung von dem Tissue-Papiersubstrat auf die Haut übertragbar ist und in einer ausreichenden Menge vorliegt, um Enzymaktivität auf der Haut zu hemmen, und wobei die Enzymhemmungszusammensetzung Hemmstoffe für Enzyme, die in menschlichem Schleim vorhanden sind, umfasst.

2. Tissue-Papierprodukt nach Anspruch 1, umfassend ein Tissue-Papiersubstrat und eine topisch aufgetragene Lotion, wobei die topisch aufgetragene Lotion die Enzymhemmungszusammensetzung umfasst.

3. Tissue-Papierprodukt nach Anspruch 3, wobei die Enzymhemmungszusammensetzung Hemmstoffe umfasst, die ausgewählt sind aus der Gruppe, bestehend aus Lipasehemmstoffen, Proteasehemmstoffen und Mischungen davon.

4. Tissue-Papierprodukt nach einem der Ansprüche 1 bis 3, wobei die Enzymhemmungszusammensetzung eine oder mehrere Esterverbindungen umfasst.

5. Tissue-Produkt nach Anspruch 2, wobei die topisch aufgetragene Lotion ferner einen Weichmacher umfasst, der ausgewählt ist aus der Gruppe, bestehend aus quartären Ammoniumverbindungen, Polysiloxanen und Mischungen davon.

6. Tissue-Papierprodukt nach einem der vorstehenden Ansprüche, wobei das Tissue-Papierprodukt eine flächenbezogene Masse von 10 bis 100 Gramm pro Quadratmeter, vorzugsweise von 25 bis 60 Gramm pro Quadratmeter aufweist.

7. Verwendung eines Enzymhemmungsmittels bei der Herstellung eines Taschentuchs zur Hemmung von Enzymaktivität auf der Haut des Nasenbereichs.

8. Verfahren zur Herstellung eines Tissue-Papierprodukts, das ein Taschentuch ist, umfassend die folgenden Schritte:
Nasslegen eines Zellstoffes zur Bildung einer Bahn, wobei der Zellstoff Fasern enthält, die im Wesentlichen aus Cellulosefasern oder von Cellulose abgeleiteten Fasern bestehen;
Trocknen der Bahn, um eine Lage eines Tissue-Papiersubstrats zu bilden;
wahlweise Kombinieren von zwei oder mehr Lagen, um das Tissue-Papiersubstrat bereitzustellen;
topisch Auftragen einer Lotion;
**dadurch gekennzeichnet, dass** die Lotion eine Enzymhemmungszusammensetzung umfasst, wobei die Enzymhemmungszusammensetzung von dem Tissue-Papiersubstrat auf die Haut übertragbar ist und in einer ausreichenden Menge vorliegt, um Enzymaktivität auf der Haut zu hemmen, und wobei die Enzymhemmungszusammensetzung Hemmstoffe für Enzyme, die in menschlichem Schleim vorhanden sind, umfasst.

9. Verfahren nach Anspruch 8, wobei die Lotion auf das Tissue-Papiersubstrat entweder gesprüht oder gedruckt wird.

## Revendications

1. Produit de papier de soie, c'est-à-dire un mouchoir pour le visage comprenant un substrat de papier de soie et une composition d'inhibition des enzymes, la composition d'inhibition des enzymes étant transférable du substrat de papier de soie vers la peau et étant présente en une quantité suffisante pour inhiber l'activité enzymatique sur la peau, et dans lequel la composition d'inhibition des enzymes comprend des inhibiteurs d'enzymes présents dans le mucus humain.

2. Produit de papier de soie selon la revendication 1, comprenant un substrat de papier de soie et une lotion appliquée de manière topique, la lotion appliquée de manière topique comprenant la composition d'inhibition des enzymes.

3. Produit de papier de soie selon la revendication 3, dans lequel la composition d'inhibition des enzymes comprend des inhibiteurs choisis parmi le groupe constitué d'inhibiteurs de lipases, d'inhibiteurs de protéase, et leurs mélanges.

4. Produit de papier de soie selon l'une quelconque des revendications 1 à 4, dans lequel la composition d'inhibition des enzymes comprend un ou plusieurs composés d'ester.

5. Produit de papier de soie selon la revendication 2, dans lequel la lotion appliquée de manière topique comprend en outre un agent adoucissant choisi parmi le groupe constitué de composés d'ammonium quaternaire, polysiloxanes et leurs mélanges.

6. Produit de papier de soie selon l'une quelconque des revendications précédentes où le produit de papier de soie a une masse surfacique comprise entre 10 et 100 grammes par mètre carré, de préférence entre 25 et 60 grammes par mètre carré.

7. Utilisation d'un agent d'inhibition des enzymes dans la fabrication d'un mouchoir pour le visage pour l'inhibition de l'activité enzymatique sur la peau de la région du nez.

8. Procédé pour la fabrication d'un produit de papier de soie, c'est-à-dire un mouchoir pour le visage comprenant les étapes consistant à :
appliquer par voie humide une pulpe pour former une nappe, la pulpe contenant des fibres constituées sensiblement par des fibres de cellulose ou des fibres dérivées de la cellulose ;
assécher la nappe pour former un pli d'un substrat de papier de soie ;
facultativement, combiner deux ou plusieurs plis pour fournir le substrat de papier de soie ;
appliquer une lotion de manière topique ;
**caractérisé en ce que** la lotion comprend une composition d'inhibition des enzymes, la composition d'inhibition des enzymes étant transférable du substrat de papier de soie vers la peau et étant présente en une quantité suffisante pour inhiber l'activité enzymatique sur la peau, et dans laquelle la composition d'inhibition des enzymes comprend des inhibiteurs d'enzymes présents dans le mucus humain.

9. Procédé selon la revendication 9, dans lequel la lotion est ou vaporisée ou imprimée sur le substrat de papier de soie.
